# EUROPEAN PATENT APPLICATION

(11) **EP 4 571 304 A1**
(43) Date of publication of application: **18.06.2025**
(21) Application number: 24219877.8
(22) Date of filing: 13.12.2024
(51) Int. Cl.: G01N 33/18, C02F 1/40, C02F 101/32

(54) **OIL CONTENT MONITORING SYSTEM AND METHOD**

(30) Priority: 13.12.2023 GB 202319039
(71) Applicant: Rivertrace Limited, Redhill Surrey RH1 4DP (GB)
(72) Inventor: Saunders, Martin, Redhill, RH1 4DP (GB); Coomber, Mike, Redhill, RH1 4DP (GB); Wickenden, Andrew, Redhill, RH1 4DP (GB); Lovering, Gillian, Redhill, RH1 4DP (GB); Draper, James, Redhill, RH1 4DP (GB)
(74) Representative: Optimus Patents Limited

(57) **Abstract**

A method and system for automatically monitoring bilge water for a vessel is described. The method comprising the steps of: receiving a bilge water sample for analysis in an Oily Water Separator; determining a status of the Oily Water Separator; determining the PPM concentration of oil in the bilge water sample at a specific date time, GPS location and vessel speed using an oil content monitor; automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for the start and end of each discharge; automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and automatically providing all recorded data for each discharge of bilge water that occurs to an electronic record book.

## Description

### Technical Field

The technical field relates to a system for monitoring bilge water in a ship and for recording information from the oil content monitor to meet necessary regulatory requirements.

### Background

All ships complying to marine regulation MEPC.107(49) must discharge bilge water to the ocean via equipment approved to meet the standards set within MEPC.107(49). This regulation came into effect on the 1^{st} January 2005 and applies to all vessel exceeding 400 gross tons that had the hull laid, or at a similar stage of construction on this date. Bilge water is a mixture of water, oil, and other contaminants that accumulate in the bilge the lowest part of a ship's hull. It comes from machinery leaks, maintenance operations, and condensation Compliance with this marine regulation ensures that ships contribute to marine conservation by protecting marine life from harmful pollution, and also reduces the risk of fines and reputational damage to ship operators.

There are three main elements to conventional bilge water discharges; an MEPC.107(49) approved **oily water separator,** an MEPC.107(49) approved **oil content monitor (OCM)** and **competent crew.** The process involves separating oil from water to ensure that the water discharged into the sea meets strict environmental standards. The OWS removes oil and other contaminants in multiple stages, including coarse separation, fine separation and polishing. The OCM continually monitors oil content of the water before it is discharged overboard. The OCM will analyse a small sample of the bilge water, for example using light scattering technology or UV fluorescence to determine the oil content. If the oil content is below threshold of 15 PPM, the bilge water can be discharged if the oil content is above the threshold, discharge is halted and at the water is diverted back for further treatment until the threshold is met.

The largest responsibility lies on the crew of the vessel. These responsibilities are very time consuming and allows numerous opportunities for human errors to occur. The OWS operates to separate the oil from the water contained in the ships bilge before it gets discharged to sea. Currently, an OCM is a device that continually monitors for the presence of oil in this discharge bilge water and will only allow the discharge into an external body of water if the actual oil content is less than 15ppm (or 5ppm in the Canadian Great lakes and certain other special areas). The discharge process (log file) must be available onboard for Port state control officials to view whenever they want to. This data must also be entered into the Oil Record Book on board. This is where mistakes occur.

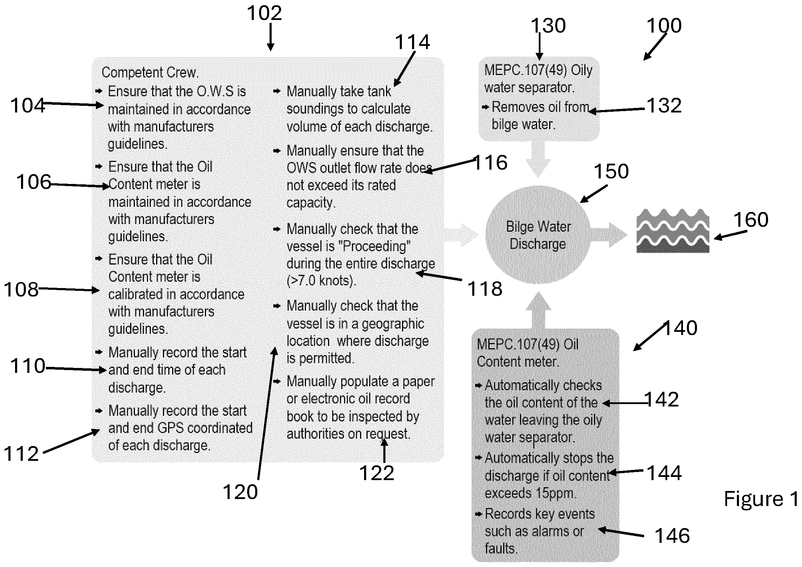

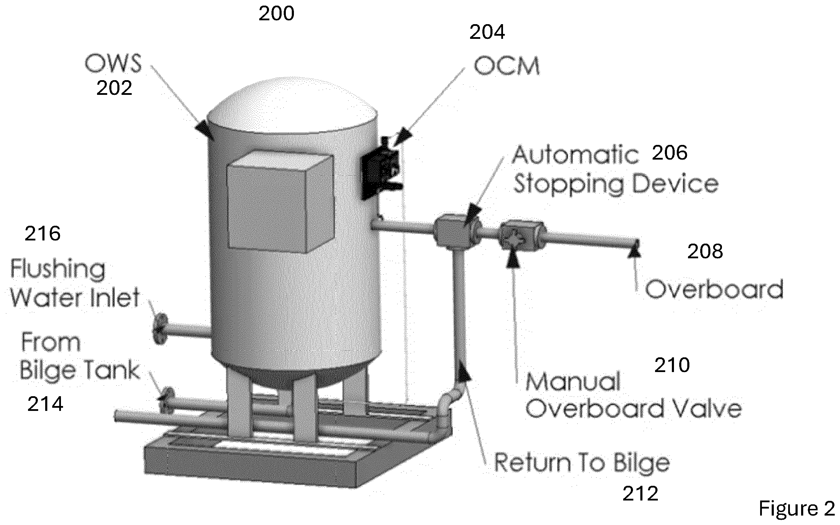

Figure 1 shows the standard process 100 currently performed using existing metering systems, and figure 2 shows an example of a known oil content metering system 200. The metering system 200 has an Oil Water Separator (OWS), 202, and oil content meter 204, automatic stopping device 206, overboard 208, manual overboard valve 210, return to bilge 212, bilge inlet 214, flushing water inlet 216. All oil content meters on the market offer the features listed in 140, regardless of manufacturer are designed to meet strict MEPC requirements. The oil content meter140 must carry out the following checks and actions.
- Generate an alarm if the water sample exceeds 15ppm (parts Per Million). To note, there are some vessels that operate under more stringent regulations which limit the maximum permissible oil content to 5ppm. This is referred to as "Clean Design".
- Generate an alarm if a fault occurs within the oil content meter.
- Generate an alarm if clean water is selected for flushing (As the monitor is no longer monitoring bilge water).
- Record every alarm or fault that takes place, alongside a GMT Time stamp.
- Record each time the Oily Water separator is Started.
- Record each time the Oily Water Separator is Stopped.
- Directly control an automatic stopping device (Valve) that prevents bilge water discharges going into the ocean if an alarm is generated.
- Generate an alarm if the monitors internal time or date is altered by the user.

The data that is recorded is referred to as "IMO Data" as this is checked and policed by the (International Maritime organisation).

Alongside the use of MEPC approved equipment, the ship's crew must also work to strict MARPOL rules when using the equipment. The rules are the following:
- The Ship is proceeding enroute.
- The bilge water has been processed by a suitably approved oily water separator (OWS).
- The oil content of the bilge water is less than 15ppm without dilution.
- The oil content of the bilge water does not originate from cargo.
- The bilge water is not contaminated with cargo oil (on board oil tankers).

All oily water discharges must then be manually recorded in an "Oil Record Book". This is a paper spreadsheet where all oily water movements are recorded. The vessel must record the following data:

### Standard IMO Data requirements:

➢ **PPM**
   o Each data entry records the PPM reading at the given timestamp,
   o Generates an alarm and prevents any bilge water discharge where the sample oil content exceeds 15ppm.
➢ **Time & Date**
   o Each data entry is time and date stamped with GMT Time.
➢ **Oily water Separator pump status**
   o Each data entry records the state of the oily water separator pump (either Pump ON or OFF).
➢ **Oil content meter faults**
   o Generates an alarm and prevents any bilge water discharge until the fault is cleared.
   ∘ Each fault that occurs is logged within the IMO data log.

MEPC Regulations require data to be stored within the oil content meter for a minimum of 18 months. This stored data is referred to as IMO data.

Process 100 also details various crew responsibilities, 102, an approved oil water separator 130, and oil content meter 140, the bilge discharge 150 and the external water body, 160, a sea, river, lake or other water body.

Crew responsibilities 102 comprise the following:
Ensuring the OWS is maintained in accordance with manufacturers guidelines, 104

Ensure that the oil content metre is maintained in accordance with manufacturers guidelines, 106.

Ensure that the oil content metre is calibrated in accordance with manufacturers guidelines, 108.

Manually record the start and end time of each discharge, 110.

Manually record the start and end GPS coordinates of each discharge, 112.

Manually takes tank soundings to calculate the volume of each discharge, 114.

Manually ensure that the OWS outflow rate does not exceed its rated capacity, 116.

Manually check that the vessel is proceeding during the entire discharge at a speed of greater than or equal to 7.0 knots, 180.

Manually check that the vessel is in a geographic location where discharge is permitted, 120.

Manually populated paper or electronic oil record book to be inspected by authorities on request, 122.

The oily water separator pump 130, removes oil from the bilge water, 132. Oil content meter 140, performs the following steps:
Automatically checks the oil content of the water leaving the oily water separator, 142

Automatically stops the discharge if oil content exceeds 15 PPM (or 5ppm in special areas), 144.

Records key events such as alarms or faults, 144.

When the crew, the oil content meter 140 and the oily water separator 130 work correctly in accordance with the necessary standards bilge water is discharged from the vessel, 150, into a body of water 160.

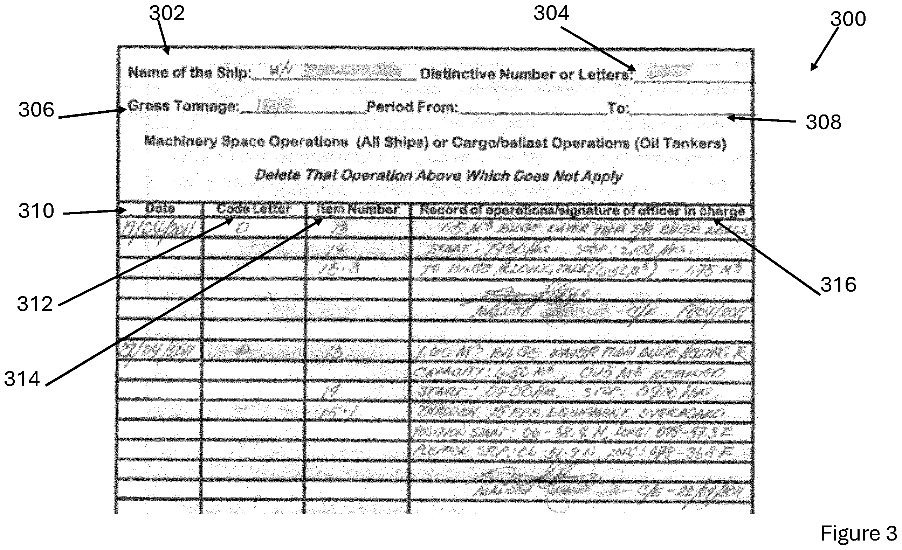

All transfers or discharges of oil or potentially oily water in the bilge water discharge 150 must be recorded in an oil record book. Figure 3 is an example of traditional paper oil record book 300: As seen, this will include the name of the ship 302, any distinctive numbers or letters 304, the gross tonnage 306, the period covered by the book 308, the date for an entry 310, the code letter for an entry 312, an item number 314 and a record of the operations 316. Historically, this is a paper spreadsheet that is completed by hand, by one or more members of the crew. With so many different elements to consider, on top of a high pressure, multilingual, technical working environment, mistakes are not uncommon. The paper oil record book is susceptible to physical damage, leading to permanent loss of records.

Legibility of the text is also problematic due to writing styles, alongside the ability to modify text at a later date, breaching MEPC guidelines which states the logbook must be correctly recorded in chronological order.

Errors, non-compliant discharges and inaccurate logbook keeping often result in substantial fines, running into hundreds of thousands and even millions of dollars. These issues will also incur additional fees as the vessels can be detained by authorities until discrepancies are resolved.

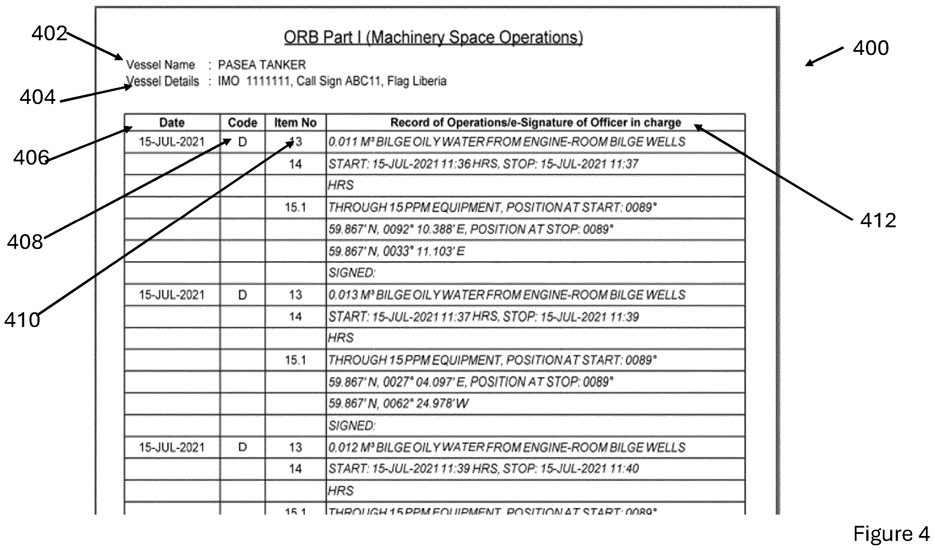

With a change in attitudes towards technology in the marine industry and advances in technological abilities, approved electronic oil record books are gaining a firm foothold. For these, the data will be manually input, but will be stored in an electronic format. An example of electronic oil record book output 400 is shown in figure 4, this includes vessel name 402, vessel details 404, date 406, item code 408, item number 410, record of operations 412. Uptake of electronic records has been is slow, however it is increasing exponentially.

Whilst the data still has to be manually entered into the record book an electronic oil record books will eliminate poor handwriting, physical logbook damage and the ability to modify the records at a later date. Some oil record books also crosscheck discharge volume data against tank levels to help limit discrepancies, only allowing plausible data to be entered, e.g. Tank level shows 10m3 of water onboard at last inventory so any new discharge data volume cannot exceed 10m3.

In practice, electronic oil record books do very little to relieve workload of the crew, increase accuracy of data or eliminate the possibility of human error, since the data still has to be manually entered into the record book.

### Summary of the Invention

According to an example there is provided a method of automatically monitoring bilge water for a vessel comprising the steps of: receiving a bilge water sample for analysis in an Oily Water Separator; determining the status of the Oily Water Separator; determining the PPM concentration of oil in the sample at a specific date time, GPS location and vessel speed using an oil content monitor; automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for the start and end of each discharge; automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; automatically providing all recorded data for each discharge of bilge water that occurs to an electronic record book.

Preferably, the recorded data further comprises details of any alarms generated, and any discharge prevention.

In a preferred embodiment, the minimum allowed speed is 7 knots.

In an example, the method further comprising the step of calibrating the oil content meter in accordance with a designated calibration schedule. Preferably, the method further comprising the step of maintaining at least one of the oily water separator and the oil content monitor in accordance with the manufacturer's instructions. In an example of the method a maintenance schedule for at least one of the oily water separator and the oil content monitor is set according to recorded parameters for the bilge water discharge.

Preferably, the recorded data is stored within a control assembly of the oil content monitor. Further preferably, the recorded data is downloadable in a read only format. In a preferred example the recorded data is downloadable as an application planning interface (API).

In an example the data provided to the electronic record book can be used to provide a discharge summary for each discharge from the vessel.

According to an example, there is also provided a system for automatically monitoring bilge water for a vessel comprising an oily water separator, a connection module, a GPS system, an electronic record book, and a connection module connecting the oily water separator to the electronic record book, the oily water separator configured to perform the steps of: receiving a bilge water sample for analysis in an Oily Water Separator; determining the status of the Oily Water Separator; determining the PPM concentration of oil in the sample at a specific date time, GPS location and vessel speed using an oil content monitor; automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for each discharge; automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and wherein the electronic record book automatically records all recorded data for each discharge of bilge water that occurs from the oily water separator.

Preferably, the GPS system is connected to the oil content meter of the oily water separator.

Further preferably, the system comprising a stopping device connected to the oily water separator, and an overboard valve downstream of and connected to the stopping device. In a preferred example, the system further comprising a flow meter located between the stopping device and the overboard valve. Preferably, the overboard valve is a manual overboard valve.

The embodiments and examples herein described provide a system and method for monitoring and recording data related to bilge water on a vessel, as described in the accompanying claims. Specific example embodiments are set forth in the dependent claims. These and other aspects will be apparent from and elucidated with reference to the embodiments described hereinafter.

### Brief description of the drawings

Further details, aspects and embodiments will be described, by way of example only, with reference to the drawings. In the drawings, like reference numbers are used to identify like or functionally similar elements. Elements in the FIG's are illustrated for simplicity and clarity and have not necessarily been drawn to scale.
FIG. 1 illustrates a simplified flow chart of know prior art process for recording bilge water discharge information;
FIG. 2 illustrates a schematic view of a prior art oil water separator and oil content meter;
FIG. 3 illustrates an example of a paper record book for bilge water discharge;
FIG. 4 illustrates an example of an electronic record related to bilge water discharge
FIG. 5 illustrates an example of a prior art oil water separator and oil content meter, with electronic record system;
FIG. 6 illustrates the processes and steps for an embodiment;.
FIG. 7(a) illustrates an example of data captures according to an embodiment;
FIG. 7(b) illustrates an example of data provided from an electronic record system to an API gateway.

### Detailed Description

The design of the bilge water monitoring system is a novel and inventive concept by the inventors of this application, that is focussed on alleviating pressure on the crew, whilst improving data accuracy and correctness. The inventors of this application are the first to develop a technical solution that records all necessary aspects of the discharge and automatically populates digital oil record books with all necessary data to satisfy any inspecting authority.

Alongside the standard MEPC.107(49) IMO requirements, as outlined above, the metering system according to an embodiment can record and/or prevent discharges relating to the following parameters:

### Additional log and control features:

**> Discharge Flow rate.**
   ∘ Measures the discharge flow rate leaving the MEPC.107(49) Oily water separator. Typically this is measures in litres/minute, and the total flow measured in litres.
   ∘ Records and totalises the volume of water that has been discharged.
   ∘ Generates an alarm and prevents any bilge water discharge that exceeds the rated capacity of the Oily Water Separator, and records details of all alarms that are generated..
➢ **Sample Flow rate**
   o Records the state of the sample flow rate.
   ∘ Generates an alarm and prevents any bilge water discharge if the sample flow rate deviates from permissible limits.
➢ **GPS Position.**
   ∘ Records the Position of the ship at each IMO data entry, including the GPS position at the start and stop of each discharge.
➢ **Ships Speed**
   o Monitors and records the speed of the vessel, utilising GPS data.
   ∘ Generates an alarm and prevents any discharge if the vessel's speed drops below the minimum legal discharge speed.
➢ **Digital output of IMO data through SmartConnect.**
   ∘ Passes a complete discharge summary to the ships digital oil record book for each discharge that takes place.

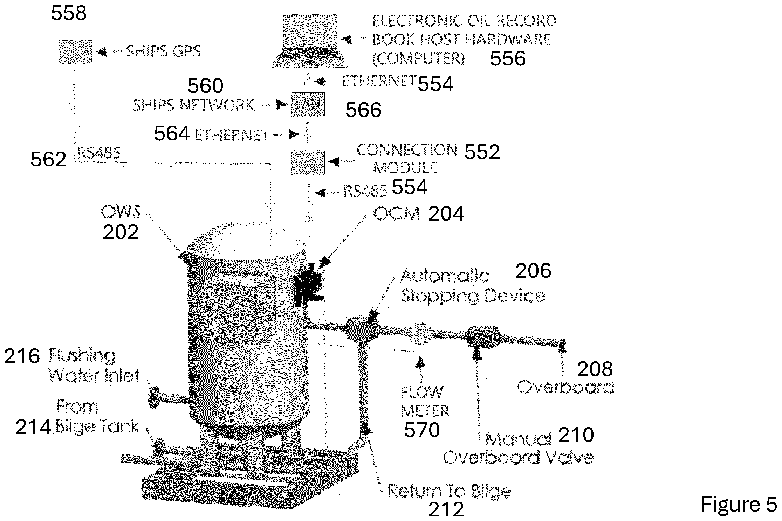

FIG. 5 is an example of a oil content monitoring system with direct connection to an electronic record system 550. The oil content monitoring system comprises an Oil Water Separator (OWS), 202, and oil content meter 204, automatic stopping device 206, overboard valve 208, manual overboard valve 210, return to bilge 212, bilge inlet 214, flushing water inlet 216. This is connected to the electronic record system 550. Preferably, there is provided a stopping device connected to the oily water separator, and an overboard valve downstream of and connected to the stopping device. In an embodiment, the oil content monitoring system further comprising a flow meter located between the stopping device and the overboard valve, preferably the overboard valve is a manual overboard valve. In an example, the OCM 204 is connected to a connection module 552 via an RS485, 554. The ship GPS system 558 is also connected to the OCM via an RS485 connection 562. Preferably, the GPS system is connected to the oil content meter of the oily water separator. The connection module 552 is connected via an ethernet connection 564to the ship network 560, preferably a LAN 566. The LAN 566 is connected via an ethernet connection 564 to a computer 556, which will host the electronic record book.

In an example, there is provided a system for automatically monitoring bilge water for a vessel comprising: an oily water separator, a connection module, a GPS system, an electronic record book, and a connection module connecting the oily water separator to the electronic record book, the oily water separator configured to perform the steps of: receiving a bilge water sample for analysis in an Oily Water Separator; determining the status of the Oily Water Separator; determining the PPM concentration of oil in the sample at a specific date time, GPS location and vessel speed using an oil content monitor; automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for each discharge; automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and wherein the electronic record book automatically records all recorded data for each discharge of bilge water that occurs from the oily water separator.

The additional parameters recorded by the monitoring system of this invention allow a discharge summary to be calculated and shared via the Connection module 552. The connection module 552 will receive all IMO data, preferably on an RS485 communication stream from the oil content monitor 204, summarise and package the received data into a file, preferably a file in the JSON format that is shared with the onboard electronic oil record book 556, in an API over an ethernet connection 564..

In digitalising this entire process, it is not possible for data discrepancies to occur between actual discharge specifics (Time, date, position, speed, discharged water volume etc) and the specific entry in the electronic oil record book 564 . Human error is largely eliminated, and burden of crew is greatly reduced.

Additional security measures are also provided according to an embodiment, at present these are additional features outside of the scope of the regulations are, a flow switch 570 in the feed line to the OCM to ensure a representative sample is always being measured. This is to avoid tampering with pipework configuration to trap a clean water sample in the measurement device, tricking it into allowing a discharge.

The data generated by the OCM 204is stored within the control assembly of the OCM. It can be downloaded in CSV format but it is hard coded on the device and cannot be manipulated.

Where a flow meter 570 is installed , we can use data from the flow meter 570 to work out total discharge and even calculate the amount of actual oil in ml that has been discharged. The flowmeter can also be used to detect cases where, say, a 1 ton separator is discharging 2 tons per hour, typically this is not possible unless someone has connected a fire hose to the inlet for dilution purposes.

For the digital output from the OCM, this can be adapted to the protocol of the receiving software/hardware is and all logs are effectively hand shaked for security (checksum).

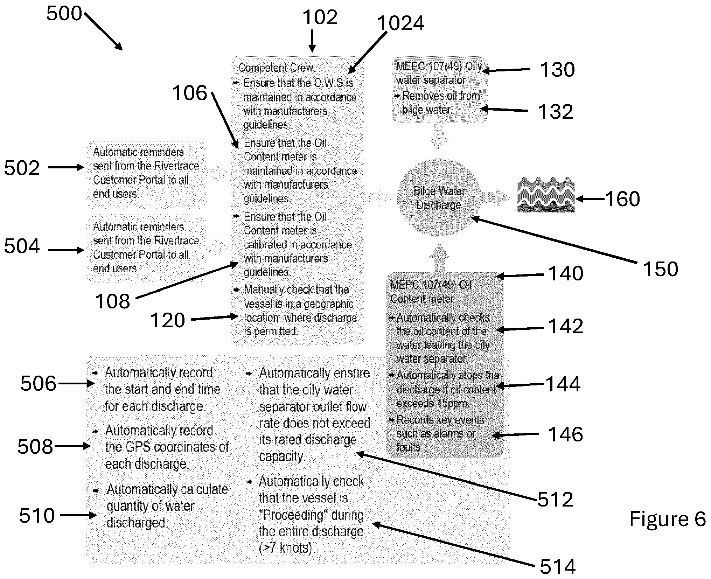

Figure 6 is an example of the steps that occur during the method 500 of an embodiment of the invention. In an example, a method of automatically monitoring bilge water for a vessel is described. The method comprising the steps of: receiving a bilge water sample for analysis in an Oily Water Separator; determining the status of the Oily Water Separator; determining the PPM concentration of oil in the sample at a specific date time, GPS location and vessel speed using an oil content monitor; automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for the start and end of each discharge; automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and automatically providing all recorded data for each discharge of bilge water that occurs to an electronic record book.

In an example, the minimum allowed speed is 7 knots.

As shown method 500 includes steps 102 taken by the competent crew, steps performed by oily water separator 130, .steps performed by oil content monitor 140. As a result of the processing steps a bilge water discharge 150, is discharged into external water body 160. The steps performed by the competent crew 102 comprise the following:
Ensuring the OWS is maintained in accordance with manufacturers guidelines, 104

Ensure that the oil content metre is maintained in accordance with manufacturers guidelines, 106.

Ensure that the oil content metre is calibrated in accordance with manufacturers guidelines, 108.

Manually check that the vessel is in a geographic location where discharge is permitted, 120.

The oily water separator pump 130, removes oil from the bilge water, 132.

The Oil content meter 140, is configured to perform one or more of the following steps, and so these are not performed by the crew:
Automatically check the oil content of the water leaving the oily water separator, 142

Automatically stops the discharge if oil content exceeds 15 PPM, 144.

Records key events such as alarms or faults, 144. Preferably, the recorded data will comprise details of any alarms generated and any discharge prevention.

When the crew, the oil content meter 140 and the oily water separator 130 worked correctly in accordance with the necessary standards bilge water is discharged from the vessel, 150, into a body of water 160.

The oil content meter 140 also automatically performs the following steps:
Automatically record the start and end time for each discharge, 506.

Automatically record the GPS coordinates of each discharge, 508.
automatically calculate the quantity of water discharged during each discharge, 510.
automatically ensure that the oily water separator outlet flow rate does not exceed its rated discharge capacity, 512.

Automatically check that the vessel is proceeding during the entire discharge at a speed greater than 7 knots, 514.

The recorded data related to one of more of the steps 506-514 is preferably stored within a control assembly of the oil content monitor. Further preferably, downloadable in a read only format. In an example, the recorded data is downloadable as an application planning interface (API). Preferably, the data provided to the electronic record book can be used to provide a discharge summary for each discharge from the vessel.

Also shown in this figure are the following external steps:
Automatic reminders sent from a customer portal to all and end users, 602, to ensure that the oil content meter is maintained in accordance with manufacturers guidelines. In a preferred example, the method further comprising the step of maintaining at least one of the oily water separator and the oil content monitor in accordance with the manufacturer's instructions.

Automatic reminders sent from a customer portal to all end users, 604, to ensure that the oil content meter is calibrated in accordance with manufacturers guidelines. Preferably, the method further comprising the step of calibrating the oil content meter in accordance with a designated calibration schedule. Preferably, the maintenance schedule for at least one of the oily water separator and the oil content monitor is set according to recorded parameters for the bilge water discharge.

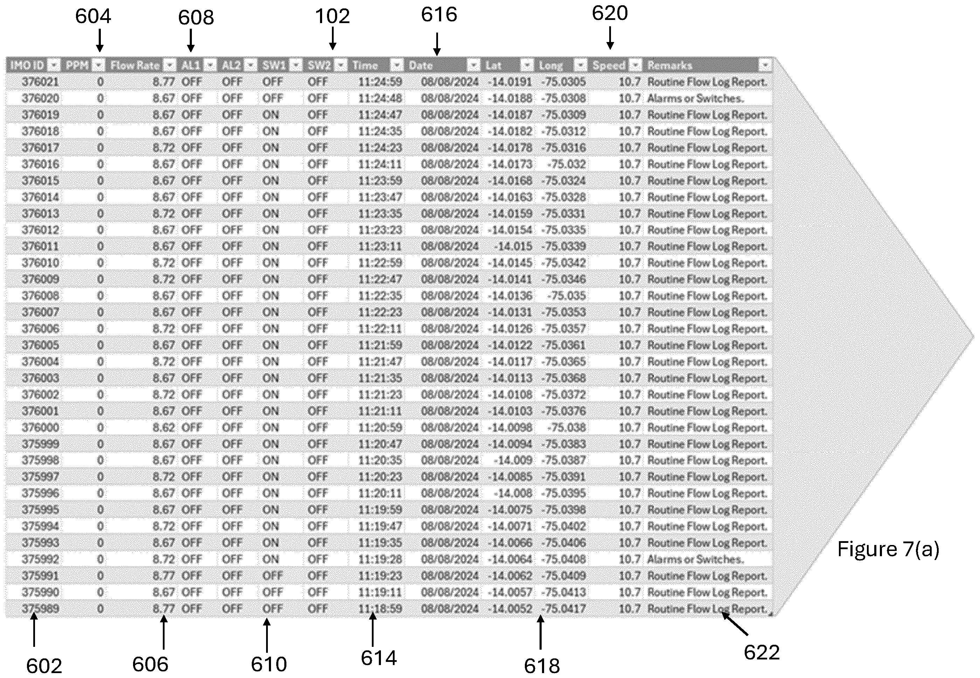

Figure 7(a) shows an example of data collected using an embodiment of the invention. This data collected includes Data log ID Number 602, PPM 604, Discharge flow rate 606, Alarm states AL1 and AL2 608, Separator state SW1 610, backflush state SW2 612, time 614, date 616 and position 618, which shows latitude and longitude, as well as speed, in knots 620 and any relevant remarks 622.

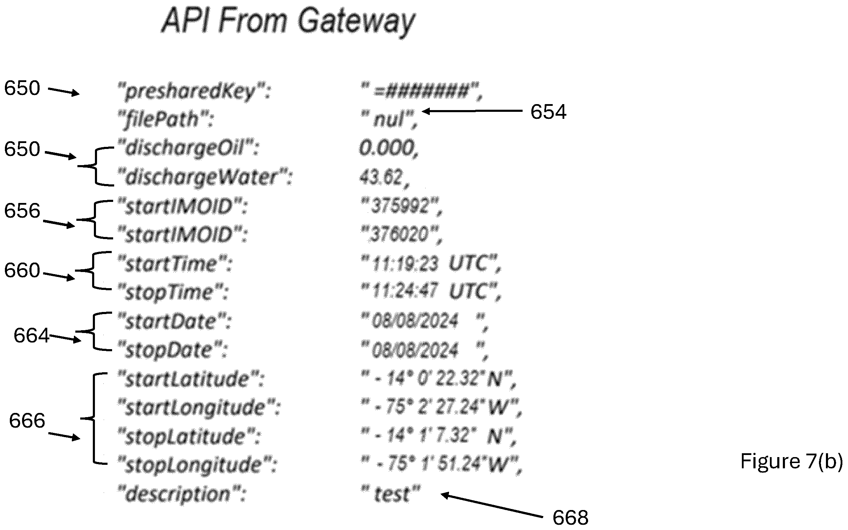

Figure 7(b) shows the information that is extracted from the record of Figure 6(a) and provided to an API, preferably by a smart connect gateway. The information includes a preshared key 650, a file path 654, discharge information 656, comprising oil discharge information and water discharge information, data log ID reference 656, timing information 660, including a start time and a stop time, date information, 664, including a start date and stop date, location information 666, including a start longitude and latitude and a stop longitude and latitude, and a description 668.

Once the discharge summary is received by the onboard electronic record book 556 software, all necessary fields of the electronic record book 556 will be prepopulated with electronically gathered and accurate discharge information. This data is then presented to the crew for final human approval, which remains a manual checkpoint to be carried out by crew under regulation.

In the foregoing specification, it will be evident to a skilled person that that various modifications and changes to the examples herein described may be made therein without departing from the scope of the invention as set forth in the appended claims, and that the claims are not limited to the specific examples described above. It is envisaged that the safety mechanism discussed herein may be utilised in any type of manufacturing machine with fixed and moving parts where a mechanism must be actuated at a set relative position of components of the mechanical safety system.

Those skilled in the art will recognize that the design of the components and the mechanical process described, depicted herein are merely exemplary, and that in fact many other configurations, architectures or process steps can be implemented that achieve the same functionality.

In the claims, any reference signs placed between parentheses shall not be construed as limiting the claim. The word 'comprising' does not exclude the presence of other elements or steps then those listed in a claim. In the claims, the word 'comprising' does not exclude the presence of other elements or steps than those listed in a claim. Furthermore, the terms 'a' or 'an,' as used herein, are defined as one or more than one. Also, the use of introductory phrases in the claims, such as 'at least one', 'one or more', etc., should not be construed to imply that the introduction of another claim element by the indefinite articles 'a' or 'an' limits any particular claim to containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

## Claims

1. A method of automatically monitoring bilge water for a vessel comprising the steps of:
receiving a bilge water sample for analysis in an Oily Water Separator;
determining a status of the Oily Water Separator;
determining the PPM concentration of oil in the bilge water sample at a specific date time, GPS location and vessel speed using an oil content monitor;
automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for the start and end of each discharge;
automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and
automatically providing all recorded data for each discharge of bilge water that occurs to an electronic record book.

2. A method as claimed in claim 1 wherein the recorded data further comprises details of any alarms generated, and any discharge prevention.

3. A method as claimed in claim 1 or claim 2 where the minimum allowed speed is 7 knots.

4. A method as claimed in any preceding claim further comprising the step of calibrating the oil content meter in accordance with a designated calibration schedule.

5. A method as claimed in any preceding claim further comprising the step of maintaining at least one of the oily water separator and the oil content monitor in accordance with the manufacturer's instructions.

6. A method as claimed in claim 5 wherein maintenance schedule for at least one of the oily water separator and the oil content monitor is set according to recorded parameters for the bilge water discharge.

7. A method as claimed in any preceding claim wherein the recorded data is stored within a control assembly of the oil content monitor.

8. A method as claimed in claim 6 wherein the recorded data is downloadable in a read only format.

9. A method as claimed in claim 8 wherein the recorded data is downloadable as an application planning interface (API).

10. A method as claimed in any preceding claim wherein the data provided to the electronic record book can be used to provide a discharge summary for each discharge from the vessel.

11. A system for automatically monitoring bilge water for a vessel comprising
an oily water separator, a connection module, a GPS system, an electronic record book, and a connection module connecting the oily water separator to the electronic record book, the oily water separator configured to perform the steps of:
receiving a bilge water sample for analysis in an Oily Water Separator;
determining the status of the Oily Water Separator;
determining the PPM concentration of oil in the sample at a specific date time, GPS location and vessel speed using an oil content monitor;
automatically recording the following parameters for the bilge water sample using data from the oil content monitor: flow rate of the bilge water sample; volume of discharged water in each discharge; total volume of discharged water; discharge flow rate from the oily water separator; start and end time of each discharge, GPS coordinates for each discharge;
automatically generating an alarm and preventing discharge of bilge water when at least one of the following conditions is met: the bilge water exceeds the rated capacity of the oil water separator; the bilge water flow rate deviates from permitted flow limits; the vessel speed drops below a minimum allowed speed for discharge of bilge water; and
wherein the electronic record book automatically records all recorded data for each discharge of bilge water that occurs from the oily water separator.

12. A system as claimed in claim 11 wherein the GPS system is connected to the oil content meter of the oily water separator.

13. A system as claimed in claim 11 or 12 further comprising a stopping device connected to the oily water separator, and an overboard valve downstream of and connected to the stopping device.

14. A system as claimed in claim 13 further comprising a flow meter located between the stopping device and the overboard valve.

15. A system as claimed in claim 13 or claim 14 wherein the overboard valve is a manual overboard valve.
